(19)

Europäisches
Patentamt
European
Patent Office
Office européen
des brevets

(11) **EP 2 296 718 B1**

(12) **EUROPEAN PATENT SPECIFICATION**

(45) Date of publication and mention
of the grant of the patent:
**27.03.2013 Bulletin 2013/13**

(21) Application number: **09755172.5**

(22) Date of filing: **24.04.2009**

(51) Int Cl.:
*A61L 27/06* (2006.01)          *A61L 27/32* (2006.01)

(86) International application number:
**PCT/TR2009/000052**

(87) International publication number:
**WO 2009/145741 (03.12.2009 Gazette 2009/49)**

(54) **CALCIUM PHOSPHATE COATING OF TI6AI4V BY A NA-LACTATE AND LACTIC ACID-BUFFERED BODY FLUID SOLUTION**

CALCIUMPHOSPHAT-BESCHICHTUNG VON TI6AI4V MIT EINEM NA-LACTAT UND MILCHSÄURE-GEPUFFERTE KÖRPERFLÜSSIGKEITS-LÖSUNG

PHOSPHATE DE CALCIUM ENROBANT DU TI6AL4V AVEC UNE SOLUTION DE LIQUIDE CORPOREL TAMPONNÉE AVEC DU LACTATE DE SODIUM ET DE L' ACIDE LACTIQUE

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **27.05.2008 TR 200803782**

(43) Date of publication of application:
**23.03.2011 Bulletin 2011/12**

(73) Proprietors:
- **Pasinli, Ahmet**
  **35100 Izmir (TR)**
- **Yuksel, Mithat**
  **35100 Izmir (TR)**
- **Havitcioglu, Hasan**
  **35100 Izmir (TR)**
- **Tas, A. Cuneyt**
  **35100 Izmir (TR)**
- **Aksoy, R. Sami**
  **35100 Izmir (TR)**
- **Celik, Erdal**
  **35100 Izmir (TR)**
- **Yildiz, Hasan**
  **35100 Izmir (TR)**
- **Toparli, Mustafa**
  **35100 Izmir (TR)**
- **Canatan, Ahmet**
  **35100 Izmir (TR)**
- **Sener, Sevil**
  **35100 Izmir (TR)**

(72) Inventors:
- **Pasinli, Ahmet**
  **35100 Izmir (TR)**
- **Yuksel, Mithat**
  **35100 Izmir (TR)**
- **Havitcioglu, Hasan**
  **35100 Izmir (TR)**
- **Tas, A. Cuneyt**
  **35100 Izmir (TR)**
- **Aksoy, R. Sami**
  **35100 Izmir (TR)**
- **Celik, Erdal**
  **35100 Izmir (TR)**
- **Yildiz, Hasan**
  **35100 Izmir (TR)**
- **Toparli, Mustafa**
  **35100 Izmir (TR)**
- **Canatan, Ahmet**
  **35100 Izmir (TR)**
- **Sener, Sevil**
  **35100 Izmir (TR)**

(74) Representative: **Iskender, Ibrahim**
**Destek Patent Inc.**
**Patent Department**
**Osmangazi Mah.**
**Tophane Ortapazar Cad.**
**Zindankapi Sok. No: 10**
**Osmangazi**
**16040 Bursa (TR)**

(56) References cited:
**EP-A1- 1 348 452**

EP 2 296 718 B1

- A. CUNEYT TAS, SARIT B. BHADURI: "Rapid coating of Ti6al4V at room temperature with a calcium phosphate solution similar to 10x simulated body fluid" JOURNAL OF MATERIALS RESEARCH, vol. 19, no. 9, 1 September 2004 (2004-09-01), pages 2742-2749, XP002591785

## Description

### Field of Invention

The main objective of this study was to investigate hydroxyapatite (HA) and calcium phosphate (CaP) coatings on Ti6Al4V substrates by using the biomimetic technique. To this purpose, a new solution was developed to coat CaP on Ti6Al4V alloy substrates. The newly formulated body fluid (Lac-SBF) contained appropriate amounts of sodium lactate (NaL) and lactic acid (HL), as well as all the other ionic constituents of the human blood plasma. The inorganic ion concentrations of the Lac-SBF solutions were identical with those of human blood plasma. The new Lac-SBF solution of this study eliminated the need for using Tris/HCl or Hepes/NaOH buffers. Prior to coating process, the substrates were chemically treated in NaOH and $NaOH+H_2O_2$ as an alternative route and heat treated at 600°C for 1 hour in air. Using HUNaL which is generated by human body and do not show any toxic behavior as buffer solution instead of tris/HCl in researches presented in the literature has been marked a new epoch in biomimetic applications. It was determined that adhesion strengths of the coatings are increased in biomimetic results of the performed *in-vivo* tests. This invention will be presented a great service to man due the fact that the metal implants which are coated with CaP in the Lac-SBF solution are used in living creatures, the implant material is strongly bonded to bond surfaces, and it shortens a patient recovery period by quickly knitting to bones. This case is understood via better bonding of CaP coated anchor screws to bone in a demonstration of biomimetic results.

### Background of Invention

Titanium alloy (Ti6Al4V) is a material which is commonly used in the manufacturing of orthopedic implants such as hip prostheses, bone plates and bone screws. The excellent mechanical properties, low density and inert nature of this material made it desirable for restoring the structural integrity of hard tissues that have undergone physical or pathogen-related trauma. The non-corrosive nature of titanium has many biological benefits. Commercially pure titanium and Ti6Al4V do not induce a negative immune response from the body and exhibit very low incidence of toxicity and rejection. Nevertheless, this same lack of interaction with the biological environment partially prevents the implant from integrating with the surrounding hard tissue (L. L. Hench, J. Am. Ceram. Soc., 1991, 74, 1487-1510).
Many methods, such as physical machining and controlled oxidation, have been used to improve the *in-vivo* osseointegration of titanium-based implants (F. Barrere, et. al., 2002, Biomaterials, 23, 1921-1930; K. C. Baker, et. al., 2006, Materials Science and Eng. C, 26, 1351-1360).
More recently, CaP coatings such as hydroxyapatite $[Ca_5(PO_4)_3(OH)]$ have been used on orthopedic implants (H. Li, et al. , Mater. Sci. Eng. C, 2007, 27, 756-761; T. Kokubo, et. al., J. Mater. Sci. Mater. M., 2004, 15, 99-107; A. Bigi, et. al., Biomaterials, 2005, 26, 4085-4089). The addition of this bioceramic to the surface of oxidized metallic implants has been shown to dramatically enhance the hard tissue integration, thereby increasing the *in-situ* mechanical stabilization (M. Demircioglu, et. al., Key Eng. Mater., 2004, 264-268, 2103-2106. This hard tissue integration and stabilization would lead to quicker patient recovery times and extended life for orthopedic implants (K.C. Baker, et. al., 2006, Materials Science and Eng. C. 26, 1351-1360).
Biocompetable materials are considered to be the most important feature in biomaterials, allow the surrounding tissue to differentiate normally and preventing undesired reactions such as infection and blood clot. Due to these requirements, in-*situ* biomimetic coating method generally is chosen for these applications. In the socalled biomimetic technique, the apatite-like CaP coating is achieved by placing the substrates to be coated into a simple biocompatible aqueous medium kept at the body temperature of 37ºC and at the blood pH of 7.4 (T. Kokubo, et. al., 2004, J. Mater. Sci. Mater. M., 15, 99-107). The calcification solutions (usually called as SBF) allow the *in situ* chemical precipitation from a metastable supersaturated solution similar in ion concentrations similar to those of human blood plasma (A. Bigi, et. al., 2005, Biomaterials, 26, 4085-4089) The need for simple clinical processes of CaP deposition has lead to the development of chemical immersion techniques, in which titanium implants are allowed to soak in a solution containing high concentrations of calcium and phosphate for a specified period of time.
The solutions commonly used for this type of coating are named as simulated or synthetic body fluids, which mimic the composition of blood plasma with respect to inorganic ions. Kokubo-SBF having a $HCO_3^-$ concentration of 4.2 mM (T. Kokubo, et. al., 2004, J. Mater. Sci. Mater. M., 15, 99-107) and Tas-SBF of 27 mM $HCO_3^-$ (D. Bayraktar, A. C. Tas, 1999, J. of The European Ceramic Society, 19, 2573-2579) are the two commonly known Tris-buffered SBFs. The pH values of these solutions were adjusted to 7.4 by using the Tris/HCl pair.
For instance, patent application with the publication number of EP1348452 A1 discloses a coating method wherein a calcium phosphate (CaP) based material is deposited on a Ti6Al4V beads in a simulated body fluid which contains tris and hydrochloric acid. However, tris/HCl leads to the problem of causing higher $Cl^-$ concentration in the medium than the $Cl^-$ value in blood plasma.
Tas and Bhaduri (A.C. Tas et. al., J. Mater. Res., 2004, 19, 2742-2749) had previously shown that it was possible to coat Ti6Al4V substrates at room temperature with an apatite-like CaP layer much more rapidly (in few hours) by using a solution having ten times the concentrations of $Ca^{2+}$ and $HPO_4^{2-}$ ions than present in a typical SBF solution, also without a need for the presence of a buffering agent, such as Tris or Hepes. Later, Bigi et al. (A. Bigi, et. al., 2005, Biomaterials, 26, 4085-4089) also demonstrated a supersaturated calcium phosphate solution, but buffered with Hepes, to maintain the pH in the vicinity of physiological levels.

X. Shen et.al. (X. Shen, et. al., 2007, Materials Letters, 61, 629-634) has developed a new *in-situ* precipitation technique for speeding up calcium phosphate nucleation and growing in polymer hydro gel. Poly acrylic acid/HA composites are prepared and used for building nano-scale structures.

N. Zhang et.al. have claimed that bone cells contain three dimensional nano composite structures and produce 45-nm diameter fibrous nanocrystal HA particles (N. Zhang et. al., 2007, Materials Science and Engineering C, 27, 599-606),

H. Li et. al. have prepared SBF by using NaCl, NaHCO3, KCl, $K_2HPO_4$, $MgCl_2$, $CaCl_2$ and $Na_2SO_4$ and added KF to have 5 ppm F level to CaP coating at pH=7.25 (H. Li et. al., 2007, Materials Science and Engineering C, 27, 756-761).

As a result, the apatite and brushite coating of Ti implants after chemical and heat treatments increased the bone-implant interface bonding strength, and thus this method was found to be advantageous for load bearing implants as demonstrated in Refs. (M. Kawashita et. al., 2003, Biomaterials 24, 2477-2484).

## Description of Invention

In this study, we exemplified a new approach in preparing solutions similar to body fluids and sodium lactate (NaL) and lactic acid (HL) were used to adjust the pH at the physiological level, without using Tris or Hepes. HL was used instead of Tris. It is important to note that while HL is present in the human body, but Tris or Hepes are not. We used high purity starting chemicals of $CaCl_2 \cdot 2H_2O$, $MgCl_2 \cdot 6H_2O$, KCl, NaCl, $Na_2HPO_4 \cdot 2H_2O$, $Na_2SO_4$, $NaHCO_3$, NaL and HL in preparing these solutions in distilled water.

The resultant Lac-SBF solutions precisely matched all of the ion concentrations of the human blood plasma. CaP coatings were formed on Ti6Al4V substrates by using these novel Lac-SBF solutions.

Upon investigating the previous methods, NaOH has been used as an etching fluid of Ti6Al4V base materials in surface finishing before coating process, but $NaOH+H_2O_2$ mixture has not yet used in literature. This invention will be provided a considerable contribution in applications with usage of $NaOH+H_2O_2$ mixture which is used in preprocessing of the substrate in application as an etching fluid, and a subsequent preparation of a rough surface which is more proper in a strong adhesion of the coatings to the substrate.

In literature studies in all biomimetic applications, it was determined that $Cl^-$ ion concentration was much higher than 103 mM which exists in human blood plasma. In addition, tris/HCL was used as a buffer solution in all studies. In this invention, **HL/NaL** buffer system, which is generated by human body and do not show any toxic behavior as a buffer solution, is used for the first time in the literature.

In the previous applications, $Cl^-$ concentration was found to be higher than that in blood plasma **(103 mM)** as a result of tris/HCl or hepes which is used to prevent precipitation and to keep the pH level at certain values. In this invention, instead of tris/HCl, **HL/NaL** was used and **$Cl^-$** concentration was kept at **103 mM** value. For the first time the prepared SBF was shown similar concentration to human blood plasma in terms of all inorganic ions.

Structural and characteristic properties and all advantages of invention will be understood by the help of the given figures and the detailed explanation addressed to those figures. Therefore evaluation should be done by considering these figures and detailed explanations.

## Detailed Description of Invention

In this invention, coating, analyses, and testing of CaP on Ti6Al4V base metal are listed as below. Here;

Figure 1. Process steps for formation of CaP phases.
Figure 2. FTIR analysis of biomimetic coatings formed on chemically pretreated Ti6Al4V after soaking in 2.5X Lac-SBF for 2 days.
Figure 3. XRD patterns of CaP coatings on Ti6Al4V alloy substrates pretreated in (a) NaOH and (b) $NaOH+H_2O_2$ solutions.
Figure4. Adhesion characteristics of the CaP coatings on Ti6Al4V substrates pretreated in NaOH and $NaOH+H_2O_2$
Figure 5. Biomechanical test results of CaP coated and uncoated anchor screws after *in-vitro* tests.

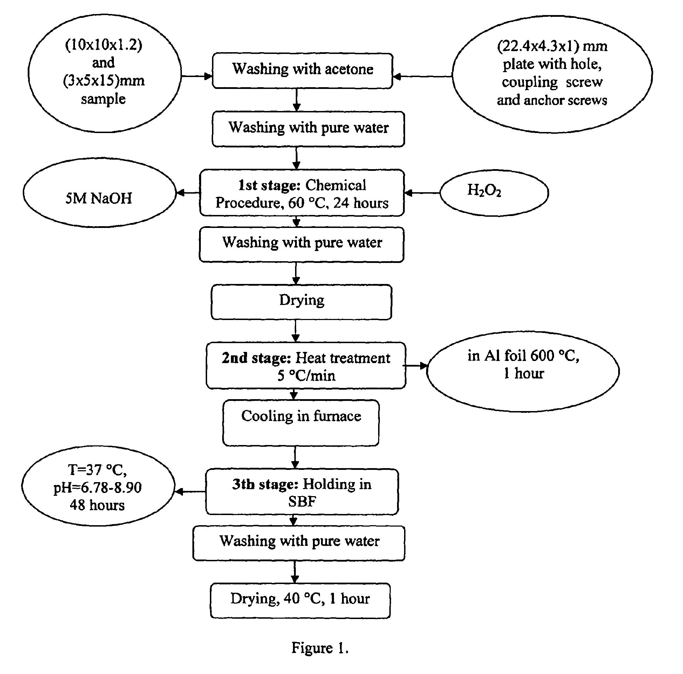

Where CaP coating was performed according to flow chart shown in Figure 1. Chemical composition of the Ti implant alloy (i.e., Ti6Al4V) used in this study was given in Table 1. Ti6Al4V specimens were first abraded with SiC papers and then cleaned with acetone and distilled water.

Table 1. Chemical composition of Ti alloy substrate

| Element content | (%) |
| --- | --- |
| Ti | Balance |
| N | 0.003 |
| C | 0.005 |
| H | <.0005 |
| Fe | 0.1 |
| O | 0.09 |
| Al | 6.21 |
| V | 3.87 |
| Y | <0.001 |
| Others | <0.3 |

Both NaOH and NaOH+$H_2O_2$ solutions were used for the chemical etching process to compare their merits after the biomimetic coating. One half of the total substrates were chemically etched in 5 M NaOH aqueous solution at 60°C for 24 h. After adding an appropriate amount of $H_2O_2$ solution to a 5 M NaOH solution, the other half of the substrates was chemically treated at 60°C for 24 h in the NaOH+$H_2O_2$ solution. All the substrates were thoroughly washed with an ample supply of distilled water, followed by drying at 40°C for 24 h in air. The etched substrates were finally heat-treated at 600°C in a microprocessor-controlled furnace with heating rates of 5°C/min, soaked at 600°C for 1 h and were then cooled slowly in the furnace down to room temperature.

Lac-SBF was prepared by using the salt amounts given in Table 2. It must be noted that the recipe of Table 2 was for preparing 2.5X Lac-SBF solutions in a total volume of 2500 mL. In preparing the 2.5X Lac-SBF solutions, only the $Ca^{2+}$ and $HPO_4^{2-}$ ion concentrations were multiplied by a factor of 2.5, with respect to those of human blood plasma. Following the addition of $CaCl_2.2H_2O$ and $MgCl_2.6H_2O$ into the solution, a total volume of 40 mL of 1 M HL was periodically added into the solution to prevent precipitation.

The substrates to be coated were placed in glass containers into a constant-temperature water bath (maintained at 37°C) and the coating time was 48 h at 37°C. Lac-SBFX2.5 solution was fed to the sample containers by a peristaltic pump at the feed rate of 4 mUh, meaning that the solution was replenished at the stated rate during the entire biomimetic coating period of 48 h at 37°C. At the end of 48 h, the coated samples were taken out of the solution and were washed with distilled water.

Finally, the samples were dried at 40°C for 24 h in air. In case of SBFx1 and SBFx2.5, only $Ca^{2+}$ and $PO_4^{3-}$ ions were used as once and 2.5 times respectively.

Table 2. Preparation of Lac-SBFX2.5 (total volume=2.5L)

| Reagents | Amount (mg) |
| --- | --- |
| $CaCl_2.2H_2O$ | 2297.3 |
| $MgCl_2.6H_2O$ | 762.5 |
| KCl | 932.5 |
| NaCl | 12053.3 |
| $Na_2HPO_4.2H_2O$ | 1112.5 |
| $Na_2SO_4$ | 177.5 |
| $NaHCO_3$ | 5670.8 |
| Na lactate (70-72%, d:1.375-1.385) | 10457.3 |
| Lactic acid (1 M) | 40.0 (mL) |

The ion concentrations of the Lac-SBF solutions were compared with those of Tris-buffered Kokubo-SBF's (T. Kokubo, et.a al., 1999, Comp. Part A: App. Sci. and Man. 30, 405-409) and Tas-SBF's (A.C. Tas, et. al., 2004, J. of Material Research, 19, 2742-2749) in Table 3. The prepared Lac-SBFx1 was shown similar concentration to human blood plasma in terms of all inorganic ions, which is the first time in the literarture.

Table 3. Comparative ion concentrations of Tris-SBFs and the Lac-SBF of this study

| Ion | Kokubo-SBF (mM) | Tas-SBF (mM) | Lac-SBFx1 (mM) | Lac-SBFx2.5 (mM) | Blood (mM) | Plasma meq/l |
|---|---|---|---|---|---|---|
| $Na^+$ | 142.0 | 142.0 | 142.0 | 142.0 | 142.0 | 142.0 |
| **Cl-** | 147.8 | 125.0 | **103.0** | **103.0** | 103.0 | 103.0 |
| $HCO_3^-$ | 4.2 | 27.0 | 27.0 | 27.0 | 27.0 | 27.0 |
| $K^+$ | 5.0 | 5.0 | 5.0 | 5.0 | 5.0 | 5.0 |
| $Mg^{2+}$ | 1.5 | 1.5 | 1.5 | 1.5 | 1.5 | 3.0 |
| **$Ca^{2+}$** | 2.5 | 2.5 | 2.5 | **6.25** | 2.5 | 5.0 |
| **$HPO_4^{2-}$** | 1.0 | 1.0 | 1.0 | **2.5** | 1.0 | 2.0 |
| $SO_4^{2-}$ | 0.5 | 0.5 | 0.5 | 0.5 | 0.5 | 1.0 |
| **Na-Lactate** | - | - | **22** | **26.5** | (+) ion:(155 meq/l or 162.5 meq/l) | |
| **Lactic acid (1M)** | - | - | 36 mL | 40 mL | (-) ion:(133 meq/l + 22 meq/l organic anions) or | |
| Tris | 50 | 50 | - | - | (136meq/l+26.5meq/ l organic anions) | |
| Ionic Str. (mM) | | | 160.5 | 173.25 | | |

Turbidity and pH value of the prepared solutions were measured.

The ionic strength of the solutions was calculated by using Equation 1.

$$I = \frac{1}{2} \sum_{i=1}^{n} c_i z_i^2$$

(1)

Where $c_i$ is the molarity concentration of ion $i$, $z_i$ is the charge of that ion, and the sum was taken over all ions present in the solution. The ionic strength was respectively calculated to be 160.5 mM and 173.25 mM for Lac-SBFx1 and Lac-SBFx2.5 solutions.

FTIR (Perkin Elmer) absorption spectra of the CaP samples were measured over the range of 4000 $cm^{-1}$ to 400 $cm^{-1}$. XRD patterns of the CaP coatings were gathered to identify the phases present with a Rigaku (D/MAX-2200/PC) diffractometer by using $CuK_\alpha$ radiation (wavelength, $\lambda$=0.15418 nm) by both $\theta$-$2\theta$ mode and $2\theta$ scan mode. Surface roughness and the thickness of the coatings were evaluated by a standard surface roughness machine (TIME, TimeSurf For TR200 V1.0) and the optic microscope.

Adhesion strength of CaP coatings were determined by using a SHIMADZU Scanning Scratch Tester (SST-W101 Model). Scratch test is carried out to determine and analyze the adhesion strength of the CaP coatings to the substrates.

Fourteen adult New Zealand white rabbits with a preoperative weight at 2500 gr were used for the *in-vivo* experiments. They were kept under standardized living conditions. Under sterile conditions the proximal part of the right tibia was bilaterally opened with a medial approach and was drilled anterior posterior into the metaphases of the rabbit with the same torque force till the whole groove was inserted. Animals were sacrificed after 6 weeks.

The biomechanical tensile tests were performed by using the testing machine (AG-I 10 kN, Shimadzu, Japanese). The mechanic testing was carried out in room temperature. A static loading was applied to the anchors. Tensile tests were performed to fourteen adult New Zealand white rabbits. Seven animals were used for each group. Seven of them were selected as an uncoated Ti6Al4V anchors (without CaP) called control group. The other seven rabbits were selected as Ti6Al4V anchors coated with CaP. The anchors were pulled-out of the right tibia of the animals. The pull-out tests were performed to the bones at a constant speed of 5mm/min. The bones were fixed to the test machine for stabilization. In all tests, the anchors were pulled out perpendicularly to the bone's surface. Anchor eyelet was pulled straight in line with the suture anchor axis, and the force at failure was reported. The differences between uncoated and coated groups in tensile test, the force data were evaluated with a Mann-Whitney U test to estimate groups' differentiations by using SPSS 11.0 for Windows.

Turbidimeter is an instrument for measuring the loss in intensity of a light beam shined through a solution that contains suspended particulate matter. Turbidimetric or nephelometric measurements were used to confirm the complete dissolution of powder-based salts in the perfectly transparent solutions. The turbidity values of the Lac-SBF solutions were given in Table 4. Their turbidity values before and after coating process was indicated that powder based chemical precursors was completely dissolved in the solutions. What we want to emphasize here that the difference between two solutions is because $Ca^{2+}$ and $PO_4^{3-}$ ions were consumed in the solutions owing to coating process. However, it can be expressed that no significant change in turbidity values were observed.

Table 4. Turbidity and pH value of solutions before and after coating process

| Solutions | Turbidity (ntu) | pH value |
| --- | --- | --- |
| Before coating | 3.315 | 6.78 |
| After coating | 0.928 | 8.90 |

The pH value of such supersaturated solutions is an important factor influencing the solubility, stability and the nucleation-growth of apatite-like CaP phases, and this should be taken into account in preparing solutions. pH value of the Lac-SBF solution should be adjusted close to 7 because precipitation easily forms in the solution when pH value increases (T. Kokubo, et.a al., 1999, Comp. Part A: App. Sci. and Man. 30, 405-409). During the coating of CaP on Ti6Al4V substrates by the biomimetic technique at 37ᵒC, the pH value of the transparent solution increased from 6.78 to around 8.9. The cause of this pH increase could be explained by the reactions of the $HPO_4^{2-}$ and $HCO_3^-$ ions in the solution with HL, as seen in Equations 2 and 3 below,

$$HPO^{2-}_4 + HL \Leftrightarrow H_2PO^-_4 + L^- \quad (2)$$

$$HCO^-_3 + HL \Leftrightarrow L^- + H_2CO_3 (=H_2O + CO_2) \quad (3),$$

Initially, $HPO_4^{2-}$ and $HCO_3^-$ were converted to about 39% $H_2PO_4^-$ and 76.5% $CO_2$, respectively. Since $HCO_3^-$ is soluble in water, $CO_2$ easily formed and escaped off of the solution during the process. Besides, the pH value increase causes a precipitation of Ca, P and $CO_3$ while preparing solutions. CaP has been deposited on Ti6Al4V substrates which were chemically cleaned with NaOH ve $NaOH+H_2O_2$ by immersing into coating solutions. The pH value increases slightly at the end of the process. Some Ca ions in the solution approach to Ti-O-Na, Al-O-Na groups which are formed on substrate surface during chemical cleaning of Ti and Al, are replaced with Na ions, and enhance the deposition of Ca-P on the surface of substrate by bonding on activated metal such as $[Al-O-Ca\ Ca_4(PO_4)_3]$, $[Ti-O-Ca\ Ca_4(PO_4)_3]$. Additionally replacement of $H^+$ ions of the water molecule with $Na^+$ ions increases alkalinity of the solution because Na ions have weak bonds to Ti-O-Na and Al-O-Na groups. This situation is explained by the increase of pH values of the solution. These chemical reactions are shown as below.

$$Ti-O-Na + H-O-H \rightarrow Ti-O-H + NaOH$$

$$Al-O-Na + H-O-H \rightarrow Al-O-H + NaOH \quad (4)$$

Increase of pH value after this reaction provides faster Ca-P coating on the surface of the substrate.

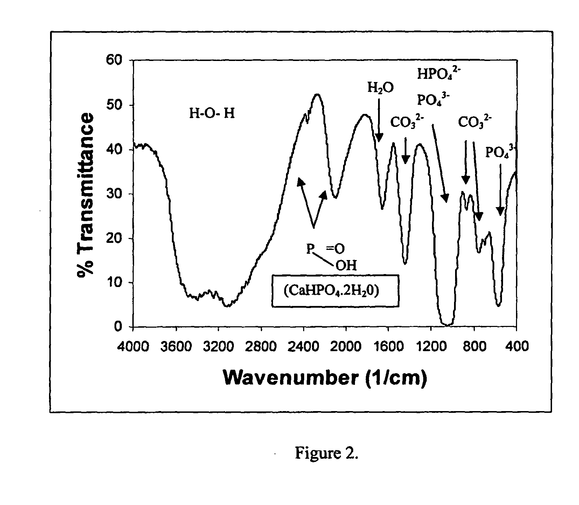

The FTIR spectra of the biomimetic CaP coatings formed after 2. days in 2.5X Lac-SBF were given in Fig. 2. A broad phosphate band originating from the P-O asymmetric stretching mode of the phosphate group was found in the region from 1100 to 1000 $cm^{-1}$, indicating a deviation of the phosphate ions from their ideal tetrahedral structure. The IR bands detected at 1460-1420 and 875 $cm^{-1}$ were assigned to the $CO_3^{2-}$ group of carbonated apatite where the $PO_4^{3-}$ groups were substituted by $CO_3^{2-}$. Additionally, the absence of the usually sharp vibration band derived from hydroxyl ions at 3500-3700 $cm^{-1}$ probably indicates the substitution of hydroxyl (OH⁻) ions by carbonate ions in the apatite structure.

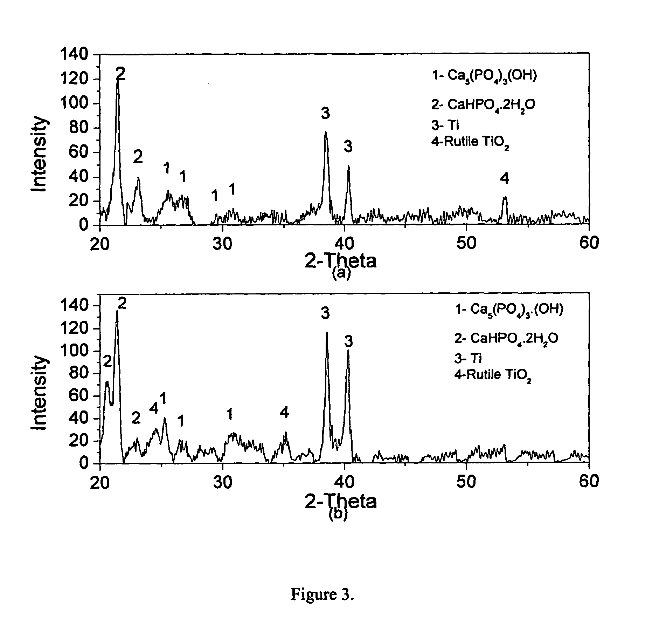

Figure 3 depicted the XRD traces of CaP coatings obtained on the Ti6Al4V alloy substrates. $Ca_5PO_4.2H_2O$ (apatite) and $CaHPO_4.2H_2O$ (brushite), titanium and rutile ($TiO_2$) phases were detected on the coatings obtained on Ti6Al4V substrates which were chemically treated in NaOH and $NaOH+H_2O_2$. Brushite was the major crystalline phase. The rutile, $TiO_2$, phase was formed on the Ti6Al4V substrates during their heat treatment at 600°C. It was also obvious from Figure 3 that the Ti peak obtained at the $2\theta$ value of 38.47° corresponded to the (002) plane.

The coating thickness and surface roughness values were reported in Table 5. 2.5X Lac-SBF solutions were able to coat a 4 $\mu$m-thick layer in 48 h on the Ti6Al4V substrates of this study. Furthermore, surface roughness values of the coatings treated in NaOH and $NaOH+H_2O_2$ were found to be 0.140 $\mu$m and 0.246 $\mu$m, respectively. In this context, it is expressed that surface roughness of CaP coatings changed depending on chemical treatment of substrate as shown in Table 5.

Table 5. Coating thickness and surface roughness of the CaP coatings

| Materials | Coating Thickness ($\mu$m) | Surface Roughness ($\mu$m) |
|---|---|---|
| Ti6A14V | - | 0.192 |
| CaP coating on NaOH treated substrates | 4 | 0.140 |
| CaP coating on NaOH+$H_2O_2$ treated substrates | 4 | 0.246 |

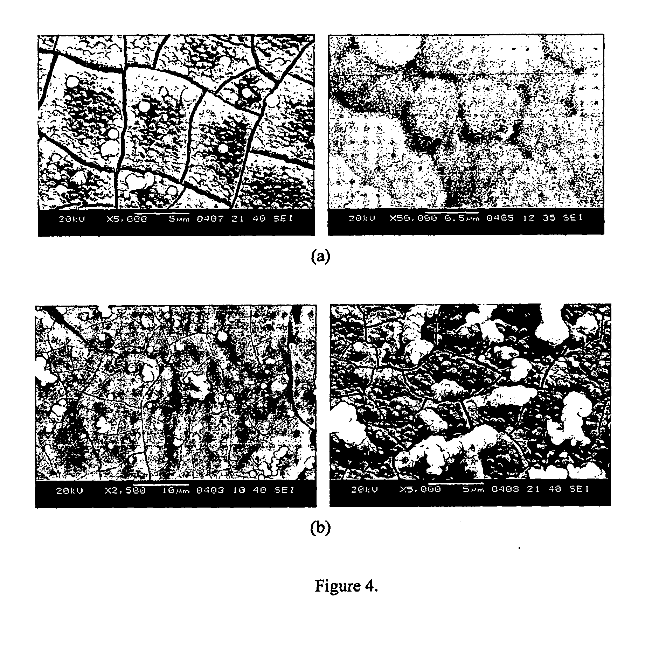

Figure 4 showed the adhesion characteristics of the CaP coatings on Ti6Al4V substrates pretreated in NaOH and NaOH+$H_2O_2$ solutions. Scratch testing is normally performed under controlled conditions (increasing load and constant speed or vice versa, or both constant) and by scratching of a coated surface with a specified indenter. A diamond-shaped indenter is usually used and from an exact knowledge of all test parameters, and by measuring the critical load at failure of the coating, the adhesion strength can be estimated (J. Forsgren, et. al., Acta Biomaterialia, 2007, 3, 980-984). In this study, the critical loads of the HA coatings treated in NaOH and NaOH+$H_2O_2$ approximately were found to be 9 mN and 3 mN, respectively. That was to say that the adhesion strength of coatings applied on Ti6Al4V surfaces pretreated in NaOH was better than those treated in NaOH+$H_2O_2$ solutions.

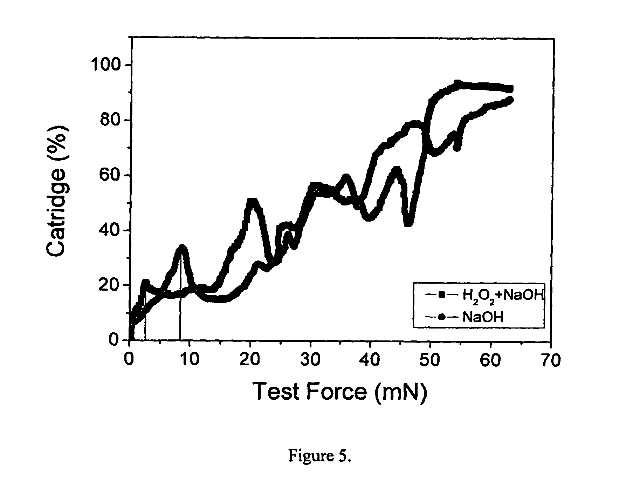

During this study, biomechanical properties of CaP coated and uncoated anchors are evaluated. The results from the current study show that CaP coated anchors have a superior mechanical performance. During the test, the load is increased until the anchors are pulled out of bone and the failure load is recorded. It has been determined that the average pull-out force of coated screws is three times of uncoated ones as given Figure 5.

In all anchors, the fixation of the anchors in test bone was stable and the anchor fixation was fairly powerful in static loading. The biomechanical data showed that a significant statistical difference was observed between the forces of Ti6Al4V anchors (without CaP) called control group and Ti6Al4V anchors coated with CaP using the Mann-Whitney test (p=0.002).

CaP coating on metal implant is an effective method to enhance bioactive properties of the metal surface. It improves the bonding strength on bone tissue without inducing the growth of fiber tissue. CaP coating can act as a barrier between the body and the metallic implant, and provide a surface on which bone can easily grow, generating mechanical interlocking and chemical bonding at the bone-implant interface.

This invention will present a great service to mankind with using the metal implants which are coated with CaP in the Lac-SBF solution in living creatures, that the implant material is strongly bonded to bond surfaces, and that it shortens a patient recovery period by quickly knitting to bones.

**Claims**

1. A modified biomimetic coating method of Calcium Phosphate (CaP) based material on Ti6Al4V surfaces of implants **wherein,** the substrate material to be coated is chemically treated in **NaOH+$H_2O_2$**, then a synthesis of CaP based (bioceramic) coating is performed using the biomimetic growth in Simulated Body Fluid (SBF), which contains appropriate amounts of **sodium lactate (NaL)** and **lactic acid (HL)** and designated as SBF with lactate (Lac-SBF).

2. A method according to claims 1, wherein, during preparation of Lac-SBF, Tris/HCl and/or Hepes/NaOH buffer solutions are not used.

3. A method according to claim 1, wherein, high purity $CaCl_2 \cdot 2H_2O$, $MgCl_2 \cdot 6H_2O$, KCl, NaCl, $Na_2HPO_4 \cdot 2H_2O$, $Na_2SO_4$, $NaHCO_3$, NaL and HL are used individually or together in distilled water for preparing Lac-SBF.

4. A method according to claim 1, wherein, the substrate materials to be coated are chemically treated in NaOH+$H_2O_2$ before they are heat treated at 600 °C for 1 hour.

5. A method according to claim 1, wherein, prior to coating and after chemically cleaning process in NaOH+$H_2O_2$, pH of the solution is set to 6.80 with 1 molar HL such that any precipitation does not form.

6. A method according to any preceding claims, wherein the Lac-SBF comprises 103,0 mM Cl[-] ion as in blood plasma.

7. A method according to any preceding claims, wherein the Lac-SBF comprises 22 mM Na-Lactat in Lac-SBFx1 at pH=7,4.

8. A method according to any preceding claims, wherein the Lac-SBF comprises 26.5 mM Na-Lactat in Lac-SBFx2.5 at pH=6,80.

9. A method according to any preceding claims, wherein, turbidity values of the solutions before coating is 3,315 ntu and/or after coating is 0,928 ntu and it reveals the completely dissolution of chemical materials in water.

**Patentansprüche**

1. Modifiziertes biomimetisches Beschichtungsverfahren von Material auf Basis von Calciumphosphat (CaP) auf Ti6Al4V-Implantatflächen, wobei das zu beschichtende Substratmaterial in $NaOH+H_2O_2$ chemisch behandelt wird, dann eine Synthese einer CaP-basierten (biokeramischen) Beschichtung mithilfe des biomimetischen Wachstums in simulierter Körperflüssigkeit (Simulated Body Fluid, SBF), die geeignete Mengen von Natriumlactat (NaL) und Milchsäure (HL) enthält und als SBF mit Lactat (Lac-SBF) bezeichnet wird, durchgeführt wird.

2. Verfahren nach Anspruch 1, wobei während der Zubereitung von Lac-SBF keine Tris/HCl- und/oder Hepes/NaOH-Pufferlösungen verwendet werden.

3. Verfahren nach Anspruch 1, wobei zur Zubereitung von Lac-SBF hochreines $CaCl_2 \cdot 2H_2O$, $MgCl_2 \cdot 6H_2O$, KCl, NaCl, $Na_2HPO_4 \cdot 2H_2O$, $Na_2SO_4$, $NaHCO_3$, NaL und HL individuell oder zusammen mit destilliertem Wasser verwendet werden.

4. Verfahren nach Anspruch 1, wobei die zu beschichtenden Substratmaterialien in $NaOH+H_2O_2$ chemisch behandelt werden, bevor sie 1 Stunde lang bei 600 °C wärmebehandelt werden.

5. Verfahren nach Anspruch 1, wobei vor dem Beschichten und nach dem chemisch Reinigen in $NaOH+H_2O_2$ der pH-Wert der Lösung mit 1 molarer HL derart auf 6,80 eingestellt wird, dass sich kein Niederschlag bildet.

6. Verfahren nach einem der vorhergehenden Ansprüche, wobei die Lac-SBF 103,0 mM $Cl^-$-Ionen wie im Blutplasma umfasst.

7. Verfahren nach einem der vorhergehenden Ansprüche, wobei die Lac-SBF 22 mM Na-Lactat in Lac-SBF x 1 bei pH=7,4 umfasst.

8. Verfahren nach einem der vorhergehenden Ansprüche, wobei die Lac-SBF 26,5 mM Na-Lactat in Lac-SBF x 2,5 bei pH=6,80 umfasst.

9. Verfahren nach einem der vorhergehenden Ansprüche, wobei die Lösungen vor dem Beschichten einen Trübungswert von 3,315 ntu und/oder nach dem Beschichten von 0,928 ntu aufweisen und der Trübungswert das vollständige Auflösen chemischer Materialien in Wasser anzeigt.

**Revendications**

1. Procédé de revêtement biomimétique modifié de matériau à base de phosphate de calcium (CaP) sur des surfaces d'implant Ti6Al4V où, le matériau du substrat à enrober est chimiquement traité dans $NaOH+H_2O_2$, et une synthèse du revêtement (biocéramique) à base de CaP est réalisée en utilisant la croissance biomimétique dans le Liquide Corporel Simulé (SBF), qui contient des volumes appropriés de lactate de sodium (NaL) et d'acide lactique (HL) et conçu comme SBF avec lactate (Lac-SBF).

2. Procédé selon la revendication 1, où, au cours de la préparation du Lac-SBF, Tris/HCl et/ou Hepes/NaOH des solutions tampon ne sont pas utilisées.

3. Procédé selon la revendication 1, où, du $CaCl_2.2H_2O$, $MgCl_2.6H_2O$, KCl, NaCl, $Na_2HPO_4.2H_2O$, $Na_2SO_4$, $NaHCO_3$, NaL et HL à pureté élevée sont utilisés individuellement ou ensemble dans une eau distillée pour la préparation du Lac-SBF.

4. Procédé selon la revendication 1, où, les matériaux du substrat sont chimiquement traités dans $NaOH+H_2O_2$ avant

d'être traités à chaud à 600 °C pendant 1 heure.

5. Procédé selon la revendication 1, où, avant le revêtement et après le processus de nettoyage chimique dans NaOH+H$_2$O$_2$, le pH de la solution est défini sur 6,80 avec 1 volume molaire HL de sorte qu'aucune précipitation ne se forme.

6. Procédé selon l'une quelconque des revendications précédentes, où le Lac-SBF comprend 103,0 mM d'ion Cl$^-$ comme dans le plasma sanguin.

7. Procédé selon l'une quelconque des revendications précédentes, où le Lac-SBF comprend 22 mM Na-Lactat dans Lac-SBF x 1 avec pH=7,4.

8. Procédé selon l'une quelconque des revendications précédentes, où le Lac-SBF comprend 26,5 mM Na-Lactat dans Lac-SBF x 2,5 avec pH=6,80.

9. Procédé selon l'une quelconque des revendications précédentes, où les valeurs de turbidité des solutions avant revêtement sont de 3,315 ntu et/ou après revêtement de 0,928 ntu et révèlent la dissolution complète des matières chimiques dans l'eau.

Figure 1.

Figure 2.

Figure 3.

(a)

(b)

Figure 4.

Figure 5.

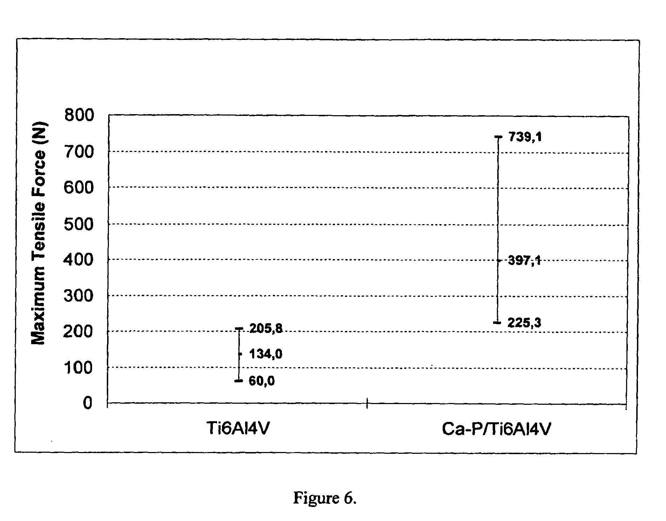

Figure 6.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1348452 A1

### Non-patent literature cited in the description

- **L. L. HENCH.** *J. Am. Ceram. Soc.,* 1991, vol. 74, 1487-1510
- **F. BARRERE.** *Biomaterials,* 2002, vol. 23, 1921-1930
- **K. C. BAKER.** *Materials Science and Eng. C,* 2006, vol. 26, 1351-1360
- **H. LI et al.** *Mater. Sci. Eng. C,* 2007, vol. 27, 756-761
- **T. KOKUBO.** *J. Mater. Sci. Mater. M.,* 2004, vol. 15, 99-107
- **A. BIGI.** *Biomaterials,* 2005, vol. 26, 4085-4089
- **M. DEMIRCIOGLU.** *Key Eng. Mater.,* 2004, vol. 264-268, 2103-2106
- **K.C. BAKER.** *Materials Science and Eng. C,* 2006, vol. 26, 1351-1360
- **D. BAYRAKTAR ; A. C. TAS.** *J. of The European Ceramic Society,* 1999, vol. 19, 2573-2579
- **A.C. TAS.** *J. Mater. Res.,* 2004, vol. 19, 2742-2749
- **X. SHEN.** *Materials Letters,* 2007, vol. 61, 629-634
- **N. ZHANG.** *Materials Science and Engineering C,* 2007, vol. 27, 599-606
- **H. LI.** *Materials Science and Engineering C,* 2007, vol. 27, 756-761
- **M. KAWASHITA.** *Biomaterials,* 2003, vol. 24, 2477-2484
- **T. KOKUBO.** *Comp. Part A: App. Sci. and Man.,* 1999, vol. 30, 405-409
- **A.C. TAS.** *J. of Material Research,* 2004, vol. 19, 2742-2749
- **J. FORSGREN.** *Acta Biomaterialia,* 2007, vol. 3, 980-984